# EUROPÄISCHE PATENTSCHRIFT

(11) **EP 3 797 813 B1**
(45) Veröffentlichungstag und Bekanntmachung des Hinweises auf die Patenterteilung: **25.10.2023**
(21) Anmeldenummer: 19199424.3
(22) Anmeldetag: 25.09.2019
(51) Int. Cl.: A61M 5/32, A61M 5/46

(54) **ZELLINJEKTIONSEINRICHTUNG, VERFAHREN ZUM ERMITTELN EINES STEUERPROGRAMMS FÜR EINE ZELLINJEKTIONSEINRICHTUNG, COMPUTERPROGRAMM UND ELEKTRONISCH LESBARER DATENTRÄGER**
CELL INJECTION DEVICE, METHOD FOR DETERMINING A CONTROL PROGRAM FOR A CELL INJECTION DEVICE, COMPUTER PROGRAM AND ELECTRONICALLY READABLE DATA CARRIER
DISPOSITIF D'INJECTION CELLULAIRE, PROCÉDÉ DE DÉTERMINATION D'UN PROGRAMME DE COMMANDE POUR UN DISPOSITIF D'INJECTION CELLULAIRE, PROGRAMME INFORMATIQUE ET SUPPORT DE DONNÉES LISIBLE ÉLECTRONIQUEMENT

(43) Veröffentlichungstag der Anmeldung: 31.03.2021
(73) Patentinhaber: Siemens Healthcare GmbH, 91052 Erlangen (DE)
(72) Erfinder: Grodzki, David, 91058 Erlangen (DE); Hengerer, Arne, 91096 Möhrendorf (DE)

(56) Entgegenhaltungen:
- WO-A1-2014/153410
- JP-A- 2009 202 331

## Beschreibung

Die Erfindung betrifft eine Zellinjektionseinrichtung zum Einbringen eines Wirkmittels in eine Zelle in vivo, aufweisend wenigstens eine Injektionskanüle für das Wirkmittel, die über einen Linearaktor in wenigstens einer Translationsrichtung translatorisch bewegbar ist. Daneben betrifft die Erfindung ein Verfahren zum Ermitteln eines Steuerprogramms zur Vornahme einer Zellinjektion mit einer Zellinjektionseinrichtung, ein Computerprogramm und einen elektronisch lesbaren Datenträger.

Im Rahmen der medizinischen und biologischen Forschung ist es bekannt, Fremdstoffe, beispielsweise ein Therapeutikum, eine Nukleinsäure, einen Zellbestandteil und/oder Nanopartikel, unter Mikroskopiekontrolle in Zielstrukturen, wie verschiedene Zellarten, Viren und Bakterien, die hier zusammenfassend als Zellen bezeichnet werden sollen, zu injizieren. Hierfür wurden im Stand der Technik bereits Mikromanipulatoren vorgeschlagen, die als Zellinjektionseinrichtung verwendet werden können. Dabei sollen im Rahmen dieser Beschreibung Viren als äquivalent zu Zellen aufgefasst werden.

Derartige Mikromanipulatoren weisen üblicherweise wenigstens eine, gegebenenfalls austauschbare, Injektionskanüle auf, über die der Fremdstoff in die jeweilige Zelle eingebracht werden kann. Die Mikrokanüle kann mittels eines Linearaktors, an den sie bewegungsgekoppelt ist, in Richtung auf die Zelle, welche durch eine Fixierungseinrichtung gehalten sein kann, bewegt werden. Die Injektionskanüle trifft dann auf die Zellwand der Zelle auf, durchbricht diese und erlaubt das Eindringen des Wirkmittels in die Zelle.

Als Zellinjektionseinrichtungen sind dabei heute Mikromanipulatoren bekannt, die eine aufwendige, teure und komplexe Mechanik aufweisen, um die gewünschten Bewegungen der Injektionskanüle umsetzen zu können.

Aus JP 2009 202 331 A ist eine Zellinjektionseinrichtung bekannt mit einer Nanopositioniereinheit und zumindest einem piezoelektrisches Element für eine Injektion.

Aus WO 2014 153 410 A1 ist eine Biopsievorrichtung zum Entnehmen mindestens einer Gewebeprobe aus einer verdächtigen Gewebemasse in einem Körper eines Lebewesens, bekannt.

Der Erfindung liegt die Aufgabe zugrunde, eine Zellinjektionseinrichtung anzugeben, die trotz des Verzichts auf eine aufwendige Mechanik einen sicher kontrollierbaren Bewegungsverlauf einer Injektionskanüle erlaubt.

Zur Lösung dieser Aufgabe ist bei einer Zellinjektionseinrichtung der eingangs genannten Art erfindungsgemäß vorgesehen, dass der Linearaktor aufweist:
- einen auf einem Gleitfilm in der Translationsrichtung bewegbar geführten Profilstab mit einem Translationsflächen aufweisenden Profil, wobei der Profilstab mit der Injektionskanüle bewegungskoppelbar ist und in einer Gleitführung gelagert ist,
- ein in zumindest einem Teil seiner möglichen Stellungen an einer Translationsfläche des Profilstabs angreifendes, drehbar gelagertes, magnetisches Pendel,
- eine Magnetfelderzeugungseinrichtung zur Erzeugung eines Magnetfelds zur Bewegung des magnetischen Pendels und
- eine Steuereinrichtung zur Ansteuerung der Magnetfelderzeugungseinrichtung derart, dass durch eine Interaktion des Pendels mit den Translationsflächen eine durch die Dimensionen der Translationsflächen und den Bewegungsweg des Pendels definierte Verschiebung des Profilstabs erfolgt.

Erfindungsgemäß wird mithin eine neuartige technische Implementierung eines linearen Aktors im Mikrometerbereich für Zellinjektionen vorgeschlagen. Diese Implementierung basiert auf der Idee eines magnetischen Pendels, das eine in einer Gleitführung, insbesondere auf einem Gleitfilm, gelagerte Mikro-Profilstruktur, nämlich den Profilstab, linear in eine Translationsrichtung schiebt. An dem Profilstab ist an einer Vorderseite, ggf. austauschbar, die Injektionskanüle befestigt, über die ein Wirkmittel, beispielsweise ein Therapeutikum, eine Nukleinsäure, ein Zellbestandteil und/oder ein Nanoteilchen, in eine Zielstruktur eingebracht werden kann. Die Zielstruktur, im Folgenden allgemein als Zelle bezeichnet, kann beispielsweise eine Eukaryontenzelle, ein Zellkompartiment, ein Bakterium oder ein Virus sein.

Das magnetische Pendel zeichnet sich nun dadurch aus, dass es durch geeignete Anregungen in insbesondere auch resonante Schwingungen versetzt werden kann. Nachdem das Pendel magnetisch ausgebildet ist, ist diese Anregung ohne eine zusätzliche Mikromechanik umsetzbar, da eine elektromagnetische Anregung mittels einer Magnetfelderzeugungseinrichtung vorgeschlagen wird. Das bedeutet, das magnetische Pendel kann ungestört frei schwingen und zieht seine Bewegungsenergie aus einem mittels der Magnetfelderzeugungseinrichtung erzeugten Magnetfelds derart, dass es in eine Wechselwirkung mit dem Profil des Profilstabs gerät, so dass eine lineare Translation in zumindest eine der beiden in der Translationsrichtung möglichen Bewegungsrichtungen resultiert. Hierfür weist das Profil des Profilsstabs Translationsflächen auf, mit denen das magnetische Pendel in dem durch das Magnetfeld der Magnetfelderzeugungseinrichtung durch Ansteuerung mittels der Steuereinrichtung herbeigeführten Bewegungsablauf interagiert.

Auf diese Weise wird ein Linearaktor geschaffen, der ohne aufwendige Mechanik aufkommt und sich durch eine besonders sichere Ansteuerung auszeichnet. Daher kann eine Injektion in eine Zielstruktur kostengünstig und ohne fehleranfällige Mechanik ausgeführt werden.

Die Strukturen des Profils des Profilstabs können in ihrer Ausdehnung dabei insbesondere im Mikrometerbereich liegen, beispielsweise zwischen 1 und 500 um. Zur Herstellung des Profilstabs können dabei Herstellungsmethoden der Mikroelektronik eingesetzt werden, beispielsweise Silizium-Ätzverfahren. Entsprechend kann der Profilstab beispielsweise aus einem typischen Mikroelektronik-Substrat bestehen.
In einer besonders vorteilhaften Ausgestaltung der vorliegenden Erfindung kann vorgesehen sein, dass der Profilstab ein Zahnstab mit mehreren, jeweils wenigstens eine Translationsfläche bereitstellenden Zähnen ist. In diesem Fall kann der Profilstab also als eine Art Mikro-Zackenstruktur verstanden werden. Jeweils wenigstens eine Seite, insbesondere eine Flanke, der Zacken beziehungsweise der Zähne kann, wie noch genauer dargelegt werden wird, als Translationsfläche dienen, mithin als eine Angriffsfläche für das Pendel, welches mit ihr interagiert und den Profilstab in seiner Gleitführung in der Translationsrichtung bewegt. Nachdem die Bewegung des Pendels im Rahmen der vorliegenden Erfindung bevorzugt periodisch erfolgt, kann jede Bewegungsperiode des Pendels zur Interaktion mit einem der Zähne des Zahnstabs führen, so dass pro Bewegungsperiode des Pendels der Zahnstab um eine entsprechende Strecke, also einen Vorschub, bewegt wird.

In diesem Kontext sieht eine erste alternative Ausgestaltung der vorliegenden Erfindung vor, dass die Zähne eine in der Drehebene des Pendels zumindest im Wesentlichen senkrecht zur Translationsrichtung stehende Translationsfläche und auf der anderen Zahnseite eine gegenüber der Translationsfläche angeordnete Abgleitfläche aufweisen, wobei das Pendel flexibel ausgebildet und/oder mit Spiel senkrecht zur Translationsrichtung gelagert ist und mittels eines Wechselfeldes der Magnetfelderzeugungseinrichtung in eine Schwingung versetzt wird. Das bedeutet, in dieser Ausgestaltung kann als periodische Bewegung des magnetischen Pendels eine Schwingung verwendet werden, deren Auslegung vorzugsweise gerade ausreicht, um den Profilstab, hier Zahnstab, "um einen Zahn" nach vorne zu versetzen. So kann das Pendel beispielsweise bei einer Hinbewegung auf die im Wesentlichen senkrecht zur Translationsrichtung verlaufende Translationsfläche treffen. In diesem Fall existieren insbesondere keine in dieser Verlaufsrichtung der Translationsfläche wirkenden Kräfte, so dass bei einer Lagerung des Pendels mit Spiel dieses Spiel nicht benutzt wird, sondern die Bewegung des magnetischen Pendels in eine Vorschubbewegung des Zahnstabs in Translationsrichtung umgesetzt wird. Bei der Rückbewegung trifft das magnetische Pendel jedoch auf die Abgleitfläche, die unter einem Winkel zu der Translationsfläche und der Translationsrichtung ausgerichtet ist, so dass eine nach oben wirkende Kraftkomponente auftritt, so dass das Spiel des Pendels in der zur Translationsrichtung senkrechten, in der Drehebene verlaufenden Erstreckungsrichtung der Translationsfläche genutzt werden kann, um sozusagen der Abgleitfläche auszuweichen und ohne einen Rückschub diese zu passieren. Nachdem das Spiel insbesondere mit einem in die Grundstellung zurückwirkenden Elastikelement versehen ist, rutscht das magnetische Pendel nach Passieren der Abgleitfläche wieder in die Grundstellung zurück und wird bei der nächsten Hinbewegung zwangsläufig auf die zugeordnete, nächste Translationsfläche treffen und den Zahnstab wiederum "um einen Zahn" nach vorne schieben.

In einer hierzu alternativen Ausgestaltung kann auch vorgesehen sein, dass die Steuereinrichtung zur Ansteuerung der Magnetfelderzeugungseinrichtung zur Erzeugung einer rotatorischen Bewegung des Pendels ausgebildet ist, welches durch Kontakt mit aufeinanderfolgenden Translationsflächen einer Ausrichtung die Linearbewegung des Profilstabs erzeugt. Auch in diesem Fall kann der Profilstab zweckmäßigerweise ein Zahnstab sein, so dass letztlich im unteren Anteil der Drehbewegung die Interaktion mit einer Translationsfläche erfolgt und der Zahnstab "um einen Zahn" weiter bewegt wird, so dass das magnetische Pendel, wenn es auf der nächsten Umdrehung zurückkehrt, an der nächsten Translationsfläche derselben Ausrichtung angreift. Dabei muss in dieser Ausgestaltung kein komplett zirkular drehendes Magnetfeld vorgesehen sein, da es ausreichend ist, wenn das Pendel im oberen Anteil der Drehbewegung hinreichend viel Schwung aufweist, um auf der anderen Seite wieder herabzufallen und dort wieder vom Magnetfeld aufgenommen und weiterbewegt zu werden. Dabei sei angemerkt, dass ein Vorteil dieser Ausgestaltung ist, dass eine gesteuerte Bewegung des Linearaktors in beide Richtungen möglich ist. So kann konkret vorgesehen sein, dass der Profilstab Translationsflächen für beide Bewegungsrichtungen der Translationsrichtung aufweist und die Steuereinrichtung zur Ansteuerung der Magnetfelderzeugungseinrichtung zur Drehung des magnetischen Pendels in beide Drehrichtungen ausgebildet ist. Bei einer Ausgestaltung als Zahnstab kann durch die Drehbewegung also an beiden Flanken der Zähne, die jeweils eine Translationsfläche bieten, angegriffen werden, je nachdem, welche Drehrichtung gewählt wird.

In konkreter Ausgestaltung der vorliegenden Erfindung kann vorgesehen sein, dass die Translationsflächen, das magnetische Pendel und der Bewegungsweg des Pendels für eine Vorschubstrecke zwischen 1 bis 500 µm, bevorzugt 5 bis 50 µm, dimensioniert sind. Wie bereits angedeutet, handelt es sich bei dem Profilstab und dem Pendel bevorzugt um Mikrostrukturen, das bedeutet, die Strukturierung liegt im Mikrometerbereich, so dass insbesondere kleine Vorschubwege nach Art eines Schrittmotors erreicht werden können. Kleine Vorschübe, insbesondere im Bereich von 5 bis 50 µm, sind erfindungsgemäß dahingehend bevorzugt, dass so ein fein abgestimmter Bewegungsablauf realisiert werden kann, der eine zu starke Beschädigung der Zielstruktur vermeidet, was wünschenswert ist. Eine möglichst genaue Steuerbarkeit ist im Rahmen der vorliegenden Erfindung beziehungsweise insgesamt bei Zellinjektionseinrichtungen wichtig, da durch unvorsichtiges oder ungenaues Durchbrechen der Zellwand die Zelle nachhaltig verletzt werden kann, woran der gesamte Vorgang scheitern kann.

Das magnetische Pendel kann konkret eine, insbesondere mittels einer über die Steuereinrichtung bestrombaren Anregungsspule induktiv bestrombare, Spule und/oder einen Permanentmagneten aufweisen oder aus einem Permanentmagneten gebildet sein. Es existieren im Rahmen der vorliegenden Erfindung also verschiedene konkrete denkbare Möglichkeiten, das magnetische Pendel zu realisieren. So kann vorgesehen sein, dass das magnetische Pendel wenigstens eine Spulenschlaufe aufweist, wobei in diese Spule beispielsweise durch eine von außen an das Pendel geführte Anregungsspule Strom induziert werden kann, so dass das Pendel eine Auslenkung erfährt. Die Auslenkung beziehungsweise der Strom in der anregenden, von außen anlegten Anregungsspule wird durch die Steuereinrichtung so gesteuert, dass das Pendel in eine periodische Bewegung, beispielsweise eine gleichmäßige Schwingung gerät.

Bevorzugt ist es im Rahmen der vorliegenden Erfindung jedoch, wenn das Pendel einen Permanentmagneten enthält beziehungsweise als ein Permanentmagnet ausgebildet ist. Dabei kann die Nord-Süd-Ausrichtung insbesondere senkrecht zur Längsachse des Pendels ausgerichtet sein. Durch Anlegen eines externen Magnetfeldes, welches durch die Magnetfelderzeugungseinrichtung erzeugt werden kann, kann auch hier das Pendel in eine periodische Bewegung versetzt werden.

In diesem Kontext kann es im Übrigen auch zweckmäßig sein, wenn die Magnetfelderzeugungseinrichtung einen über einen Aktor um dieselbe Drehachse wie das Pendel drehbaren, parallel zu dem Pendel ausgerichteten und angeordneten Permanentmagneten aufweist. In diesem Fall kann beispielsweise der zusätzliche Permanentmagnet das Magnetfeld zumindest teilweise erzeugen, durch welches das Pendel in eine insbesondere periodische Bewegung versetzt wird.

In einer demgegenüber bevorzugten Ausgestaltung der vorliegenden Erfindung ist jedoch vorgesehen, dass die Magnetfelderzeugungseinrichtung wenigstens eine Gradientenspule zur Erzeugung eines Magnetfeldgradienten aufweist. Im Rahmen der vorliegenden Erfindung hat es sich gezeigt, dass für die Magnetfelderzeugungseinrichtung eine Magnetfeldspulenkonfiguration verwendet werden kann, die ähnlich einer Endstufe einer Gradientenspulenanordnung einer Magnetresonanzeinrichtung ausgelegt ist. Für derartige Gradientenspulenanordnungen ist es auch bekannt, dass diese im Bereich von Millisekunden ihre Polarität ändern können und Felder im Millitesla-Bereich generieren können, wie sie auch im Rahmen der vorliegenden Erfindung benötigt werden. Das bedeutet, technisch bereits bekannte Ausgestaltungen für Gradientenspulen einer Magnetresonanzeinrichtung können auch auf die hier verwendete Magnetfelderzeugungseinrichtung übertragen werden.

In einer besonders vorteilhaften Ausgestaltung der vorliegenden Erfindung kann vorgesehen sein, dass der Profilstab Translationsflächen unterschiedlicher Größe und/oder unterschiedlichen Abstands, insbesondere bei der Verwendung von Zähnen Zähne unterschiedlicher Höhe und/oder Länge und/oder unterschiedlichen Abstands, aufweist, denen jeweils unterschiedliche Vorschübe in Translationsrichtung zugeordnet sind. Mit anderen Worten kann beispielsweise vorgesehen sein, dass die Zähne eines Zahnstabes eine nicht konstante Größe besitzen. Auf diese Weise kann der Abstand zwischen einzelnen Translationsflächen zu- oder abnehmen, je weiter sich der Profilstab in eine Bewegungsrichtung der Translationsrichtung bewegt, wobei es besonders bevorzugt ist, wenn die Größe der Translationsflächen und somit der zugeordneten Vorschübe in Translationsrichtung von der Injektionskanüle weg abnimmt. Auf diese Weise kann mithin bei gleichbleibender periodischer Bewegung des Pendels dennoch die Geschwindigkeit der Bewegung vorteilhaft gesteuert werden und insbesondere auch verhindert werden, dass der Profilstab sich ungewünscht weit vorbewegt, da durch den veränderten Abstand eine andere Frequenz des Pendels für eine Vorwärtsbewegung derselben Geschwindigkeit benötigt würde.

Mit anderen Worten kann durch über die Länge des Profilstabs veränderliche Translationsflächen und somit veränderliche durch die Bewegung des Pendels verursachte Vorschübe bei zeitlich und räumlich gleichartiger Bewegung des magnetischen Pendels ein Geschwindigkeitsprofil entlang des Ausfahrweges der Injektionskanüle umgesetzt werden, welches insbesondere der Aufgabe, eine Zellwand (beziehungsweise Zielstrukturwand) zu durchdringen, ohne einen zu starken Schaden zu verursachen, angepasst sein kann. Denkbar ist es beispielsweise, zunächst eine schnelle Bewegungsgeschwindigkeit der Injektionskanüle vorzusehen, bis die Nähe der Zellwand erreicht wird, dann langsamer, aber stetig, weiterzubewegen und den Druck zu erhöhen, um sicher penetrieren zu können. Auch andere Ausgestaltungen sind, je nach verwendeter Injektionskanüle und Art der Zielstruktur, selbstverständlich denkbar.

Gerade im Hinblick auf solche geplanten Bewegungswege mit zugeordneten Geschwindigkeitsverläufen und deren Umsetzung, die selbstverständlich auch über entsprechende Anpassungen der Bewegungsfrequenz des Pendel ermöglicht werden können, kann es jedoch auch allgemein zweckmäßig sein, wenn die Zellinjektionseinrichtung ferner eine Fixierungseinrichtung für eine zu behandelnde Zelle aufweist, wobei die Fixierungseinrichtung bevorzugt positionsfest zu der Gleitführung angeordnet ist. Auf diese Weise kann eine definierte Anordnung von Zielstruktur und Injektionskanüle/Profilstab in einem Grundzustand geschaffen werden, auf den sich beispielsweise Steuerprogramme, die in der Steuereinrichtung verwendet werden, um bestimmte Bewegungsabläufe herzustellen, beziehen können.

So sieht, allgemein gesagt, eine Ausgestaltung der vorliegenden Erfindung vor, dass die Steuereinrichtung zur Ansteuerung der Magnetfelderzeugungseinrichtung gemäß einem, insbesondere durch einen Algorithmus der künstlichen Intelligenz ermittelten, Steuerprogramm zur Vornahme einer Zellinjektion ausgebildet ist. Das bedeutet, in der Steuereinrichtung können, bevorzugt für unterschiedliche Anwendungsfälle und Zielstrukturen, Steuerprogramme abgelegt sein oder ermittelt werden, die ein möglichst beschädigungsfreies Injizieren in die Zielstruktur, beispielsweise ein Zellkompartiment oder ein Virus, erlauben. Die Steuerprogramme basieren dabei bevorzugt auf Vorwissen, welches durch vorangehende Versuche gewonnen werden kann. Dabei kann besonders bevorzugt auch künstliche Intelligenz eingesetzt werden.

Eine durch künstliche Intelligenz gestützte Steuerung der erfindungsgemäßen Zellinjektionseinrichtung kann bevorzugt automatisch das Durchbrechen der Kanüle in die Zellwand steuern und die Bewegung des Pendels so anpassen, dass eine nachhaltige Verletzung der Zelle mit erhöhter Sicherheit vermieden werden kann. Daher kann das Durchbrechen der Zellwand in einer Lernphase erlernt werden, indem eine hinreichend große Menge an Trainingsprozeduren durchgeführt wird. Für jede der Trainingsprozeduren sind die entscheidenden Randbedingungen, beschrieben durch Eingangsparameter, bekannt. Zu diesen Eingangsparametern und den verwendeten Bewegungsabläufen wird, beispielsweise mittels eines Mikroskops, beobachtet, wie der Durchbruch der Zellwand verläuft, so dass bewertet werden kann, wie das Durchdringen der Zellwand ablief, beispielsweise "optimal", "nicht optimal" oder "schädigend", wobei selbstverständlich auch weitere/ähnliche Kategorien zur Bewertung denkbar sind. Die dabei entstehenden Trainingsdatensätze werden verwendet, um Steuerprogramme für den späteren Betrieb der Zellinjektionseinrichtung zu lernen.

So betrifft die vorliegende Erfindung neben der Zellinjektionseinrichtung auch ein Verfahren zum Ermitteln eines Steuerprogramms zur Vornahme einer Zellinjektion mit einer Zellinjektionseinrichtung der erfindungsgemäßen Art unter Verwendung eines Algorithmus der künstlichen Intelligenz, aufweisend folgende Schritte:
- Bereitstellen mehrerer Trainingsdatensätze, welche einen Eingangsparametersatz, einen einen Bewegungsablauf der Injektionskanüle beschreibenden Bewegungsdatensatz und eine eine Bewertung des Bewegungsdatensatzes hinsichtlich der Zellverletzung beschreibende Bewertungsinformation umfassen, wobei der Eingangsparametersatz wenigstens einen die Zelle, insbesondere die Elastizität der Zellwand, und wenigstens einen die verwendete Injektionskanüle beschreibenden Eingangsparameter aufweist,
- Verwendung von maschinellem Lernen, insbesondere Deep Learning, mit dem Algorithmus der künstlichen Intelligenz zur Ermittlung einer Zuordnungsvorschrift von Eingangsparametersätzen zu Steuerprogrammen, die einen die Zellverletzung minimierenden Bewegungsablauf beschreiben.

Daneben betrifft die Erfindung auch ein Computerprogramm, welches die Schritte eines erfindungsgemäßen Verfahrens durchführt, wenn es auf einer Recheneinrichtung ausgeführt wird, und einen elektronisch lesbaren Datenträger, auf dem ein erfindungsgemäßes Computerprogramm gespeichert ist.

Der Bewegungsdatensatz kann in einer konkreten Ausgestaltung auch ein Steuerprogramm selbst sein, welches ja die Art der entstehenden Bewegung aufgrund der bekannten Geometrie der Zellinjektionseinrichtung hinreichend genau wiedergibt. Beispielsweise kann der Bewegungsdatensatz auf diese Weise beschreiben, welche Magnetfelder in welcher Geschwindigkeit von der Magnetfelderzeugungseinrichtung erzeugt werden und dergleichen. Der Bewegungsdatensatz kann jedoch auch den konkreten Bewegungsablauf wiedergeben, beispielsweise aufgrund einer angesprochenen Beobachtung durch Mikroskopie beziehungsweise mit einer Kamera. Auch hiervon ausgehend ist es selbstverständlich möglich, aufgrund der bekannten geometrischen Gegebenheiten der Zelleninjektionseinrichtung eine Umrechnung in entsprechende Steuerparameter für die Magnetfelderzeugungseinrichtung herzuleiten, die als Steuerprogramm benutzt werden können. Die Bewertung kann nach objektiven Bewertungszahlen und/oder durch einen Benutzer erfolgen.

Als Zuordnungsvorschrift kann bei wenigen, diskreten Wertkombinationen für die Eingangsparameter, beispielsweise nur eine bestimmte Anzahl von zu verwendenden Zelltypen und/oder Injektionskanülen, eine Zuordnungstabelle bestimmt werden und in der Steuereinrichtung der Zellinjektionseinrichtung eingespeichert werden. Bevorzugt und allgemeiner ist es jedoch, den trainierten Algorithmus der künstlichen Intelligenz zu verwenden, der die Zuordnungsvorschrift abbildet, nachdem er, mit Eingangsparametern versorgt, ein entsprechendes Steuerprogramm als Ausgangsparameter liefert.

Mit anderen Worten kann eine Recheneinrichtung ein zweckmäßiges Durchbrechen der Zellwand in einer Lernphase erlernen, indem sie mit einer hinreichend großen Menge an Trainingsdatensätzen aus Trainingsprozeduren versorgt wird. Wesentliche Eingangsparameter umfassen dabei insbesondere die Elastizität der Zellwand, die auch beispielsweise durch den Typ der Zelle (beispielsweise Eukaryontenzelle, Bakterium, Virus oder dergleichen) bestimmt sein kann. Ferner sind die Art und Dicke der Kanüle sowie deren Spitze wichtige Eingangsparameter. Zusammen mit dem Bewegungsdatensatz, der den Bewegungsablauf beschreibt, und beispielsweise einen verwendeten Bewegungsablauf des Pendels und/oder verwendete Magnetfeldabläufe enthalten kann, und einer entsprechenden Bewertung können die Trainingsdatensätze genutzt werden, um in einer Lernphase einen Algorithmus der künstlichen Intelligenz, insbesondere durch Deep Learning, zu trainieren. In der folgenden Anwendungsphase wird das gelernte Wissen in Form der Zuordnungsvorschrift so angewandt, dass je nach vorliegenden Eingangsparametern eine vorteilhafte Steuerung des magnetischen Pendels verwendet wird, die eine Schädigung der Zelle oder weitere den Ablauf des Verfahrens störende Fehler vermeidet. Auf diese Weise berücksichtigt die vorliegende Erfindung auch die gegebene hohe Empfindlichkeit des Vorgangs einer Zellinjektion, da durch unvorsichtiges oder ungenaues Durchbrechen der Zellwand die Zelle nachhaltig verletzt werden kann.

Durch diese Ausgestaltungen wird der vorgestellte, mechanisch wenig aufwendige Linearaktor durch eine besonders sichere, durch künstliche Intelligenz gestützte Steuerung ergänzt.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine erste Ausführungsform einer erfindungsgemäßen Zellinjektionseinrichtung,
- Fig. 2: eine zweite Ausführungsform einer erfindungsgemäßen Zellinjektionseinrichtung, und
- Fig. 3: einen Ablaufplan zum erfindungsgemäßen Verfahren.

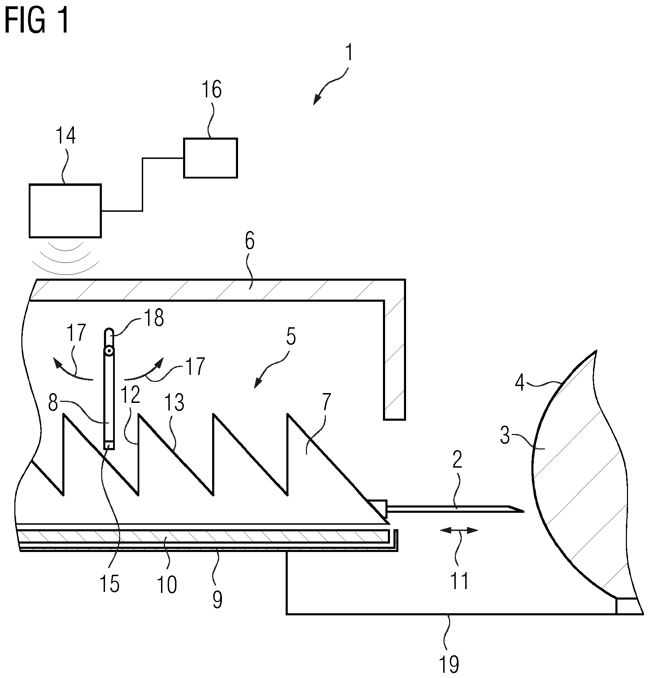

Fig. 1 zeigt eine schematische Prinzipskizze einer ersten Ausführungsform einer erfindungsgemäßen Zellinjektionseinrichtung 1. Diese soll zum Einführen einer Injektionskanüle 2 in eine hier nur angedeutete Zielstruktur, also eine Zelle 3, durch deren Zellwand 4 dienen. Hierzu ist die Kanüle mit einem Linearaktor 5 verbunden, der als in einem Gehäuse 6 befindliche Komponenten einen Profilstab 7, hier einen Zahnstab, aufweist, der mit einem drehbar gelagerten magnetischen Pendel 8 wechselwirkt. Der Profilstab 7 ist in einer Gleitführung 9, welche vorliegend einen Gleitfilm 10 aufweist, linear beweglich in einer Translationsrichtung 11 gelagert. Der Profilstab 7 weist als Profil aufeinanderfolgende Zähne auf, die jeweils an einer ihrer Flanken eine Translationsfläche 12 und an der gegenüberliegenden Flanke eine Abgleitfläche 13 aufweisen.

Durch eine Magnetfelderzeugungseinrichtung 14, die vorliegend wenigstens eine Gradientenspule, wie man sie auch von Magnetresonanzeinrichtungen kennt, aufweist, kann ein Magnetfeld erzeugt werden, in welchem sich das vorliegend einen Permanentmagneten 15 aufweisende magnetische Pendel 8 bewegt. Die Magnetfelderzeugungseinrichtung 14 kann dabei beispielsweise zylinderartig das Gehäuse 6 umgeben oder sogar bilden. Sie wird gesteuert von einer Steuereinrichtung 16, vorliegend derart, dass das Pendel 8, wie durch die Pfeile 17 angedeutet, eine Schwingbewegung mit Hin- und Rückanteilen durchführt.

Zur weiteren Erläuterung ist das Pendel 8 mit einem Spiel 18 gelagert, und zwar konkret gegen die elastische Kraft eines Rückstellmittels, welches in eine untere Grundstellung wirkt.

Trifft das Pendel 8 bei der Hinbewegung auf eine Translationsfläche 12, treten nur vernächlässigbare Kräfte in der Drehebene des Pendels 8 senkrecht zur Translationsrichtung 11 auf, und das Pendel 8 schiebt die Profilstange 7 durch Angriff an der Translationsfläche 12 und somit die Injektionskanüle 2 auf die Zellwand 4 zu. Bei der Rückbewegung jedoch trifft das Pendel 8 auf die Abgleitfläche 13, wobei auch Kräfte derart entstehen, dass das Spiel 18 benutzt wird und keine oder nur vernachlässigbare Rückbewegung auftritt. Bei der nächsten Hinbewegung greift das Pendel 8 dann an der nächsten, benachbarten Translationsfläche 12 an. So wird der Profilstab 7 in jeder Schwingungsperiode des Pendels 8 um die Länge eines der Zähne vorwärts bewegt.

Die insgesamte Vorschubgeschwindigkeit kann durch die Schwingungsfrequenz des Pendels 8 steuerbar sein. Beispielsweise kann die Dimensionierung des Pendels 8 beziehungsweise der Pendelbewegung und der Angriffsflächen 12/des Zahnabstands, allgemein also des Pendels 8, der Pendelbewegung und des Profils des Profilstabs 7, so gewählt sein, dass in jeder Schwingungsperiode ein Vorschub im Bereich von 5 bis 50 µm erfolgt.

Es sei angemerkt, dass neben einer Anpassung der Schwingungsfrequenz des Pendels 8 durch entsprechende Ansteuerung der Magnetfelderzeugungseinrichtung 14 eine Geschwindigkeitsanpassung bei gleichbleibender Schwingungsfrequenz des Pendels 8 auch dadurch erzielt werden kann, dass sich die Dimensionierung des Profils des Profilstabs 7 über seine Länge verändert, beispielsweise die Abstände zwischen den Translationsflächen 12 geringer oder größer werden, so dass pro Schwingungsperiode ein kleinerer oder größerer Vorschub gegeben ist. Hierbei wird im Allgemeinen auch eine Anpassung der Translationsflächen 12 selbst vorgenommen werden. Auf diese Art und Weise lässt sich letztlich ein Bewegungsablauf, insbesondere hinsichtlich der Geschwindigkeit, in den Profilstab 7 hineincodieren. Die ist besonders dann zweckmäßig, wenn eine definierte Ausgangsposition bezüglich der Zelle 3 gegeben ist, diese also beispielsweise mittels einer zum Gehäuse 6 und somit der Gleitführung 9 ortsfeste Fixierungseinrichtung 19 für die Zelle 3 verwendet wird.

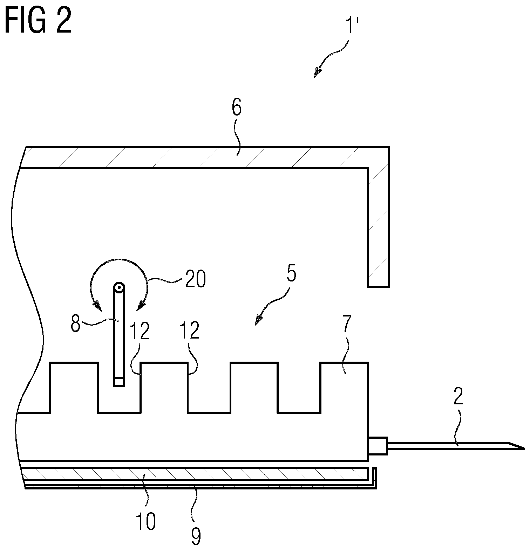

Fig. 2 zeigt eine Prinzipskizze veränderter Komponenten bei einer zweiten Ausführungsform einer erfindungsgemäßen Zellinjektionseinrichtung 1'. Dabei sind gleichwirkende Merkmale der Einfachheit halber mit gleichen Bezugszeichen versehen. Im Gegensatz zum Ausführungsbeispiel der Fig. 1 ist bei der Zellinjektionseinrichtung 1' der Profilstab 7 wieder als Zahnstab ausgebildet, weist allerdings vorliegend im Wesentlichen rechteckige Zähne auf, die an beiden Flanken eine Translationsfläche 12 aufweisen. Das Pendel 8 ist ohne Spiel vollständig rotierbar, vgl. Pfeil 20, gelagert, so dass es mittels der Magnetfelderzeugungseinrichtung 14 bei kompletter Drehung den Zahnstab durch Angriff an einer Translationsfläche 12 vorschiebt und in der nächsten Periode auf die benachbarte Translationsfläche 12 derselben Ausrichtung einwirkt. Die Steuereinrichtung 16 ist in diesem Fall dazu ausgebildet, die Magnetfelderzeugungseinrichtung 14 für beide Drehrichtungen des Pendels 8 anzusteuern, so dass eine Bewegung des Profilstabs 7 in beiden Richtungen möglich ist. Dies wird durch die Doppelpfeilnatur des Pfeils 20 ausgedrückt.

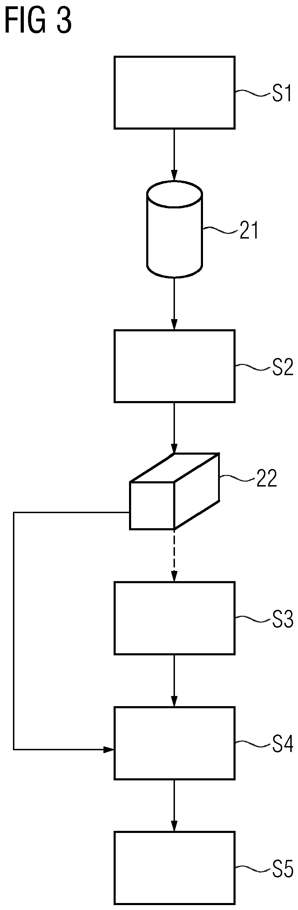

Fig. 3 zeigt eine Möglichkeit zur Vorbereitung einer sicheren, durch künstliche Intelligenz gestützten Steuerung der Zellinjektionseinrichtung 1, 1' sowie zu deren Anwendung.

Dabei werden in einem Schritt S1 mehrere Trainingsprozeduren durchgeführt, für die jeweils ein Eingangsparametersatz, ein Bewegungsdatensatz und eine Bewertungsinformation, zusammengefasst als Trainingsdatensatz, aufgenommen werden. Die Eingangsparameter beschreiben dabei die Art der verwendeten Zielstruktur, also Zelle 3, insbesondere auch die Elastizität von deren Zellwand 4. Ferner beschreibt wenigstens ein Eingangsparameter die verwendete Injektionskanüle 2, insbesondere hinsichtlich der Spitze, der Dicke und der Länge. Der Bewegungsdatensatz beschreibt den verwendeten Bewegungsablauf der Injektionskanüle 2, beispielsweise anhand von Steuerparametern eines verwendeten Steuerprogramms, oder aber auch durch die tatsächlich stattfindenden Bewegungen und ihre Geschwindigkeit. Die gesamte Trainingsprozedur wird mikroskopisch überwacht, um das Verhalten der Zellwand 4 und der Zelle 3 beobachten zu können. Die Bewertungsinformation gibt entsprechend wieder, ob der Bewegungsablauf hinsichtlich einer möglichen Beschädigung der Zelle 3 optimal, nicht optimal oder sogar schädigend war. Auch andere Bewertungsskalen sind selbstverständlich denkbar.

Die resultierende Menge von Trainingsdatensätzen 21 wird in einem Schritt S2 genutzt, um einen Algorithmus 22 der künstlichen Intelligenz derart zu trainieren, dass für einen Eingangsparametersatz ein hinsichtlich der Beschädigung der Zelle 3 möglichst optimaler Satz von Steuerparametern, also ein Steuerprogramm, ermittelt werden kann.

Der trainierte Algorithmus 22 der künstlichen Intelligenz bildet letztlich eine Zuordnungsvorschrift von Eingangsparametern auf Steuerprogramme dar. Es sei angemerkt, dass es durchaus denkbar ist, diese Zuordnungsvorschrift mit Hilfe von Maschinenlernen auch als Zuordnungstabelle oder dergleichen zu formulieren.

Nach den vorbereitenden Schritten S1 und S2 wird der resultierende Algorithmus 22 in der Steuereinrichtung 16 gespeichert und kann nun dort genutzt werden. Werden in Schritt S3 bestimmte Eingangsparameter der Steuereinrichtung 16 bereitgestellt, kann dieser in einem Schritt S4 den Algorithmus 22 der künstlichen Intelligenz nutzen, um ein geeignetes Steuerprogramm zu ermitteln. Das derart ausgewählte Steuerprogramm wird in einem Schritt S5 dann genutzt, um die Magnetfelderzeugungseinrichtung 14 anzusteuern und den durch das Steuerprogramm beschriebenen, möglichst sicher zu keinen Schädigungen der Zelle 3 führenden Bewegungsablauf der Injektionskanüle 2 mittels des Pendels 8 und des Profilstabes 7 zu erzielen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Zellinjektionseinrichtung (1, 1') zum Einbringen eines Wirkmittels in eine Zelle (3) in vivo, aufweisend wenigstens eine Injektionskanüle (2) für das Wirkmittel, die über einen Linearaktor (5) in wenigstens einer Translationsrichtung (11) translatorisch bewegbar ist, wobei der Linearaktor (5) aufweist:
- ein auf einem Gleitfilm in der Translationsrichtung (11) bewegbar geführter Profilstab (7) mit einem Translationsflächen (12) aufweisenden Profil, wobei der Profilstab (7) mit der Injektionskanüle (2) bewegungskoppelbar ist und in einer Gleitführung (9) gelagert ist, **dadurch gekennzeichnet, dass** der Linearaktor (5) weiterhin aufweist:
- ein in zumindest einem Teil seiner möglichen Stellungen an einer Translationsfläche (12) des Profilstabs (7) angreifendes, drehbar gelagertes, magnetisches Pendel (8),
- eine Magnetfelderzeugungseinrichtung (14) zur Erzeugung eines Magnetfelds zur Bewegung des magnetischen Pendels (8) und
- eine Steuereinrichtung (16) zur Ansteuerung der Magnetfelderzeugungseinrichtung (14) derart, dass durch eine Interaktion des Pendels (8) mit den Translationsflächen (12) eine durch die Dimensionen der Translationsflächen (12) und den Bewegungsweg des Pendels (8) definierte Verschiebung des Profilstabs (7) erfolgt, wobei
- die Steuereinrichtung (16) zur Ansteuerung der Magnetfelderzeugungseinrichtung (14) zur Erzeugung einer rotatorischen Bewegung des Pendels (8) ausgebildet ist, welches durch Kontakt mit aufeinanderfolgenden Translationsflächen (12) einer Ausrichtung die Linearbewegung des Profilstabs (7) erzeugt.

2. Zellinjektionseinrichtung (1, 1') nach Anspruch 1, **dadurch gekennzeichnet, dass** der Profilstab (7) ein Zahnstab mit mehreren, jeweils wenigstens eine Translationsfläche (12) bereitstellenden Zähnen ist.

3. Zellinjektionsrichtung nach Anspruch 2, **dadurch gekennzeichnet, dass** die Zähne eine in der Drehebene des Pendels (8) zumindest im Wesentlichen senkrecht zur Translationsrichtung (11) stehende Translationsfläche (12) und auf der anderen Zahnseite eine gegenüber der Translationsfläche (12) angeordnete Abgleitfläche (13) aufweisen, wobei das Pendel (8) flexibel ausgebildet und/oder mit Spiel (18) senkrecht zur Translationsrichtung (11) gelagert ist und mittels eines Wechselfeldes der Magnetfelderzeugungseinrichtung (14) in eine Schwingung versetzt wird.

4. Zellinjektionseinrichtung (1, 1') nach einem der vorangehenden Ansprüche, **dadurch gekennzeichnet, dass** der Profilstab (7) Translationsflächen (12) für beide Bewegungsrichtungen der Translationsrichtung (11) aufweist und die Steuereinrichtung (16) zur Ansteuerung der Magnetfelderzeugungseinrichtung (14) zur Drehung in beide Drehrichtungen ausgebildet ist.

5. Zellinjektionseinrichtung (1, 1') nach einem der vorangehenden Ansprüche, **dadurch gekennzeichnet, dass** die Translationsflächen (12) und der Bewegungsweg des Pendels (8) für eine Vorschubstrecke zwischen 1 bis 500, bevorzugt 5 bis 50, um dimensioniert ist.

6. Zellinjektionseinrichtung (1, 1') nach einem der vorangehenden Ansprüche, **dadurch gekennzeichnet, dass** die Magnetfelderzeugungseinrichtung (14) einen über einen Aktor um dieselbe Drehachse wie das Pendel (8) drehbaren, parallel zu dem Pendel (8) ausgerichteten und angeordneten Permanentmagneten aufweist.

7. Zellinjektionseinrichtung (1, 1') nach einem der vorangehenden Ansprüche, dass die Magnetfelderzeugungseinrichtung (14) wenigstens eine Gradientenspule zur Erzeugung eines Magnetfeldgradienten aufweist.

8. Zellinjektionseinrichtung (1, 1') nach einem der vorangehenden Ansprüche, **dadurch gekennzeichnet, dass** das Pendel (8) eine, insbesondere mittels einer über die Steuereinrichtung (16) bestrombaren Anregungsspule induktiv bestrombare, Spule und/oder einen Permanentmagneten (15) aufweist oder aus einem Permanentmagneten (15) gebildet ist.

9. Zellinjektionseinrichtung (1, 1') nach einem der vorangehenden Ansprüche, **dadurch gekennzeichnet, dass** der Profilstab (7) Translationsflächen (12) unterschiedlicher Größe und/oder unterschiedlichen Abstands, insbesondere bei der Verwendung von Zähnen Zähne unterschiedlicher Höhe und/oder Länge und/oder unterschiedlichen Abstands, aufweist, denen jeweils unterschiedliche Vorschübe in Translationsrichtung (11) zugeordnet sind.

10. Zellinjektionseinrichtung (1, 1') nach Anspruch 9, **dadurch gekennzeichnet, dass** die Größe der Translationsflächen (12) und somit der zugeordneten Vorschübe in Translationsrichtung (11) von der Injektionskanüle (2) weg abnimmt.

11. Zellinjektionseinrichtung (1, 1') nach einem der vorangehenden Ansprüche, **dadurch gekennzeichnet, dass** sie ferner eine Fixierungseinrichtung (19) für eine zu behandelnde Zelle (3) aufweist, wobei die Fixierungseinrichtung (19) bevorzugt positionsfest zu der Gleitführung (9) angeordnet ist.

12. Zellinjektionseinrichtung (1, 1') nach einem der vorangehenden Ansprüche, **dadurch gekennzeichnet, dass** die Steuereinrichtung (16) zur Ansteuerung der Magnetfelderzeugungseinrichtung (14) gemäß einem, insbesondere durch einen Algorithmus (22) der künstlichen Intelligenz ermittelten, Steuerprogramm zur Vornahme einer Zellinjektion ausgebildet ist.

## Claims

1. Cell injection facility (1, 1') for introducing an agent into a cell (3) in vivo, having at least one injection cannula (2) for the agent, which can be moved in a translational manner in at least one translation direction (11) by way of a linear actuator (5), wherein the linear actuator (5) has:
- a profile rod (7), with a profile having translation surfaces (12), which is movably guided on a sliding film in the translation direction (11), wherein the profile rod (7) can be coupled in terms of movement with the injection cannula (2) and mounted in a sliding guide (9), **characterised in that** the linear actuator (5) further has:
- a magnetic pendulum (8) which is mounted rotatably and engages in at least one part of its possible positions on a translation surface (12) of the profile rod (7),
- a magnetic field generation facility (14) for generating a magnetic field in order to move the magnetic pendulum (8) and
- a control facility (16) for actuating the magnetic field generation facility (14) such that by means of an interaction of the pendulum (8) with the translation surfaces (12), a displacement of the profile rod (7) defined by the dimensions of the translation surfaces (12) and the movement path of the pendulum (8) is carried out, wherein
- the control facility (16) is embodied to actuate the magnetic field generation facility (14) for generating a rotational movement of the pendulum (8), which generates the linear movement of the profile rod (7) by contact with consecutive translation surfaces (12) of an alignment.

2. Cell injection facility (1, 1') according to claim 1, **characterised in that** the profile rod (7) is a toothed rod with several teeth providing at least one translation surface (12) in each case.

3. Cell injection facility according to claim 2, **characterised in that** the teeth have a translation surface (12) which is at least essentially at a right angle to the translation direction (11) in the rotation plane of the pendulum (8) and on the other tooth side have a sliding surface (13) arranged facing the translation surface (12), wherein the pendulum (8) is embodied to be flexible and/or is mounted with play (18) at a right angle to the translation direction (11) and is made to oscillate by means of an alternating field of the magnetic field generation facility (14) .

4. Cell injection facility (1, 1') according to one of the preceding claims, **characterised in that** the profile rod (7) has translation surfaces (12) for both movement directions of the translation direction (11) and the control facility (16) is embodied to actuate the magnetic field generation facility (14) in order to rotate in both directions of rotation.

5. Cell injection facility (1, 1') according to one of the preceding claims, **characterised in that** the translation surfaces (12) and the movement path of the pendulum (8) is dimensioned for an advance stretch between 1 to 500, preferably 5 to 50 µm.

6. Cell injection facility (1, 1') according to one of the preceding claims, **characterised in that** the magnetic field generation facility (14) has a permanent magnet which can rotate above an actuator about the same axis of rotation as the pendulum (8) and is aligned and arranged parallel to the pendulum (8).

7. Cell injection facility (1, 1') according to one of the preceding claims, that the magnetic field generation facility (14) has at least one gradient coil for generating a magnetic field gradient.

8. Cell injection facility (1, 1') according to one of the preceding claims, **characterised in that** the pendulum (8) has a coil and/or a permanent magnet (15) which can be inductively powered in particular by means of an excitation coil which can be powered by way of the control facility (16) or is formed from a permanent magnet (15).

9. Cell injection facility (1, 1') according to one of the preceding claims, **characterised in that** the profile rod (7) has translation surfaces (12) of a different size and/or different spacing, in particular when teeth are used, teeth of a different height and/or length and/or different spacing, to which different feeds in the translation direction (11) are assigned in each case.

10. Cell injection facility (1, 1') according to claim 9, **characterised in that** the size of the translation surfaces (12) and thus the assigned feeds tapers away from the injection cannula (2) in the translation direction (11).

11. Cell injection facility (1, 1') according to one of the preceding claims, **characterised in that** it further has a fixing facility (19) for a cell (3) to be treated, wherein the fixing facility (19) is preferably arranged fixed in terms of position with respect to the sliding guide (9).

12. Cell injection facility (1, 1') according to one of the preceding claims, **characterised in that** the control facility (16) is embodied to actuate the magnetic field generation facility (14) according to a control program, determined in particular by an artificial intelligence algorithm (22) for the purpose of performing a cell injection.

## Revendications

1. Dispositif (1, 1') d'injection cellulaire pour l'injection d'un agent actif dans une cellule (3) in vivo, comportant au moins une canule (2) d'injection de l'agent actif, qui, par un actionneur (5) linéaire, peut être déplacée en translation dans au moins une direction (11) de translation, dans lequel l'actionneur (5) linéaire comporte :
- une barre (7) profilée guidée sur un film de glissement, de manière à pouvoir se déplacer dans la direction (11) de translation et ayant un profil comportant des surfaces (12) de translation, dans lequel la barre (7) profilée peut être accouplée, en déplacement, à la canule (2) d'injection et est montée dans un guidage (9) glissant, **caractérisé en ce que** l'actionneur (5) linéaire comporte en outre :
- un pendule (8) magnétique monté tournant attaquant, dans au moins l'une de ses positions possibles, une surface (12) de translation de la barre (7) profilée,
- un dispositif (14) de production de champ magnétique pour la production d'un champ magnétique pour le déplacement du pendule (8) magnétique, et
- un dispositif (16) de commande pour la commande du dispositif (14) de production d'un champ magnétique, de manière à obtenir, par une interaction du pendule (8) avec les surfaces (12) de translation, un déplacement, défini par la dimension des surfaces (12) de translation et le trajet de déplacement du pendule (8), de la barre (7) profilée, dans lequel
- le dispositif (16) de commande est constitué pour la commande du dispositif (14) de production d'un champ magnétique pour la production d'un mouvement de rotation du pendule (8), qui produit, par contact avec des surfaces (12) de translation successives d'une orientation, le déplacement linéaire de la barre (7) profilée.

2. Dispositif (1, 1') d'injection cellulaire suivant la revendication 1, **caractérisé en ce que** la barre (7) profilée est une barre dentée ayant plusieurs dents, donnant respectivement au moins une surface (12) de translation.

3. Dispositif d'injection cellulaire suivant la revendication 2, **caractérisé en ce que** les dents ont une surface (12) de translation dans le plan de rotation du pendule (8), au moins sensiblement perpendiculairement à la direction (11) de translation, et une surface (13) de glissement disposée de l'autre côté de la dent, par rapport à la surface (12) de translation, dans lequel le pendule (8) est souple et/ou monté avec jeu (18) perpendiculairement à la direction (11) de translation et est mis en oscillation au moyen d'un champ alternatif du dispositif (14) de production d'un champ magnétique.

4. Dispositif (1, 1') d'injection cellulaire suivant l'une des revendications précédentes, **caractérisé en ce que** la barre (7) profilée a des surfaces (12) de translation pour les deux sens de déplacement de la direction (11) de translation et le dispositif (16) de commande est constitué pour la commande du dispositif (14) de production d'un champ magnétique pour la rotation dans les deux sens de rotation.

5. Dispositif (1, 1') d'injection cellulaire suivant l'une des revendications précédentes, **caractérisé en ce que** les surfaces (12) de translation et le chemin de déplacement du pendule (8) sont dimensionnés pour une dimension d'avance comprise entre 1 et 500, de préférence entre 5 et 50 µm.

6. Dispositif (1, 1') d'injection cellulaire suivant l'une des revendications précédentes, **caractérisé en ce que** le dispositif (14) de production d'un champ magnétique a un aimant permanent pouvant, par un actionneur, tourner autour du même axe de rotation que le pendule (8) et dirigé et disposé parallèlement au pendule (8) .

7. Dispositif (1, 1') d'injection cellulaire suivant l'une des revendications précédentes, dans lequel le dispositif (14) de production d'un champ magnétique a au moins une bobine de gradient pour la production d'un gradient d'un champ magnétique.

8. Dispositif (1, 1') d'injection cellulaire suivant l'une des revendications précédentes, **caractérisé en ce que** le pendule (8) a une bobine pouvant être alimentée en courant inductivement, en particulier au moyen d'une bobine d'excitation pouvant être alimentée en courant par le dispositif (16) de commande et/ou un aimant (15) permanent ou est formé d'un aimant (15) permanent.

9. Dispositif (1, 1') d'injection cellulaire suivant l'une des revendications précédentes, **caractérisé en ce que** la barre (7) profilée a des surfaces (12) de translation de dimensions différentes et/ou à des distances différentes, en particulier lors de l'utilisation de dents de hauteurs différentes et/ou de longueurs différentes et/ou à des distances différentes, auxquelles sont associées respectivement des avances différentes dans la direction (11) de translation.

10. Dispositif (1, 1') d'injection cellulaire suivant la revendication 9, **caractérisé en ce que** la dimension des surfaces (12) de translation et ainsi des avances associées dans la direction (11) de translation diminue lorsque l'on s'éloigne de la canule (2) d'injection.

11. Dispositif (1, 1') d'injection cellulaire suivant l'une des revendications précédentes, **caractérisé en ce qu'**il a en outre un dispositif (19) d'immobilisation d'une cellule (3) à traiter, le dispositif (19) d'immobilisation étant disposé de préférence d'une manière fixe en position par rapport au guidage (9) à glissement.

12. Dispositif (1, 1') d'injection cellulaire suivant l'une des revendications précédentes, **caractérisé en ce que** le dispositif (16) de commande est, pour effectuer une injection dans une cellule, constitué pour la commande du dispositif (14) de production d'un champ magnétique suivant une programme de commande déterminé, en particulier par un algorithme (22) de l'intelligence artificielle.
